# FASCICULE DE BREVET EUROPEEN

(11) **EP 2 188 071 B1**
(45) Date de publication et mention de la délivrance du brevet: **29.07.2015**
(21) Numéro de dépôt: 08805609.8
(22) Date de dépôt: 21.05.2008
(51) Int. Cl.: B09C 1/10, C02F 3/34, C02F 101/32, C02F 101/34, C02F 101/38

(54) **PROCÈDE DE TRAITEMENT BACTÉRIEN D'EFFLUENTS CONTENANT DU 2-ETHYLHEXYL NITRATE**
VORRICHTUNG ZUR BAKTERIELLEN BEHANDLUNG VON ABWASSER ENTHALTEND 2-ETHYLHEXYLNITRAT
METHOD FOR THE BACTERIAL TREATING OF EFFLUENTS CONTAINING 2-ETHYLHEXYL NITRATE

(30) Priorité: 25.05.2007 FR 0703808
(43) Date de publication de la demande: 26.05.2010
(73) Titulaire: IFP Energies nouvelles, 92852 Rueil-Malmaison Cedex (FR); Commissariat A L'Energie Atomique, 75752 Paris Cedex 15 (FR)
(72) Inventeur: NICOLAU, Elodie, F-38100 Grenoble (FR); MARCHAL, Rémy, F-78400 Chatou (FR); JOUANNEAU, Yves, F-38360 Sassenage (FR)
(86) Numéro de dépôt international: PCT/FR2008/000718
(87) Numéro de publication internationale: WO 2009/004154

(56) Documents cités:
- FR-A- 2 790 752

## Description

### Domaine de l'invention:

La présente invention est relative à un procédé de traitement d'effluents comprenant du 2-éthylhexyl nitrate (ou 2-EHN) par utilisation de microorganismes capables de dégrader ce contaminant.

La contamination des sols et aquifères par les produits pétroliers et leurs dérivés est un problème important et la connaissance de la biodégradabilité de ces polluants organiques représente un enjeu évident.

Le procédé selon l'invention s'applique particulièrement aux industries du traitement de l'eau et des sols contaminés par le gazole.

### Examen de l'art antérieur :

Les nitrates d'alkyles sont ajoutés aux gazoles commerciaux afin de garantir leur indice de cétane (Suppes et al., Energy & Fuels 15, 151-157, 2001). L'indice de cétane est une caractéristique d'auto-inflammabilité des carburants diesel. Selon les motoristes une valeur de 50 de l'indice de cétane constitue une valeur minimale acceptable pour que la combustion soit contrôlée de façon satisfaisante. Dans le cas des moteurs à injection directe, l'indice de cétane conditionne également le bruit délivré par le moteur (Guibet J.C. et al. Carburants et Moteurs , Editions Technip, Paris (1999)).

Le 2-EHN est l'additif procétane le plus communément utilisé. Liquide à température ambiante, ce composé se décompose de façon autocatalytique au dessus de 100°C, libérant des radicaux libres qui réduisent le délai d'auto-inflammation du moteur. Le 2-EHN est incorporé aux carburants à hauteur de 300 à 1000 mg/L, ce qui explique que 10⁵ tonnes de ce composé sont produites annuellement dans le monde Le 2-EHN est l'additif procétane le plus communément utilisé.

Le 2-EHN est l'ester nitrique d'un alcool ramifié, le 2-éthylhexanol. Sa structure est la suivante :

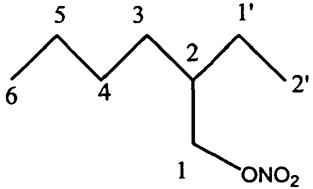

Ses principales propriétés physico-chimiques sont présentées en Tableau 1.

**Tableau 1 : Principales propriétés physico-chimiques du 2-EHN***

| Caractéristique | Valeur |
|---|---|
| N° CAS | 27247-96-7 |
| Masse molaire | 175,2 g |
| Masse volumique | 0,96 kg/L |
| Pression de vapeur à 20 °C | 27 Pa |
| Solubilité à 20 °C | 12,6 mg/L |
| Log K_{O/W} | 5.24 |

| | |
|---|---|
| *Selon "the American Chemistry Council Petroleum Additives Panel Health, Environmental, and Regulatory Task Group", octobre 2006 | |

Le gazole est biodégradable à 88% par des microflores de sols pollués par des hydrocarbures (Penet et al. Appl. Microbiol. Biotechnol., 66, 40-47, 2004). Cependant l'acceptabilité du 2-EHN est une question d'actualité surtout dans la cadre de la directive européenne REACH (Chemical Consulting Global Laboratory Network). En effet, aucune donnée concernant les propriétés de biodégradabilité du 2-EHN n'est disponible.

En revanche, les propriétés d'écotoxicité du 2-EHN sont connues. La toxicité aiguë du 2-EHN est faible puisque les LC50 (concentrations léthales 50%) vis-à-vis du poisson Zebra et de la daphnie sont supérieures à sa solubilité dans l'eau. Cependant, le 2-EHN est beaucoup plus dangereux quand il est administré à doses répétées. Chez le rat, le niveau auquel aucun effet défavorable n'est observé après 28 jours (No Observed Adverse Effect Level ou NOAEL) n'est que de 28 mg/kg.jour (Someroja et al., Toxicol. Lett, 19, 189-93, 1983). Ce résultat montre que la biodégradabilité du 2-EHN est un paramètre clé pour le risque global en cas de libération accidentelle. En effet, la biodégradabilité détermine le temps d'exposition critique à la molécule dangereuse.

Le test de Sturm (Strum, Journal of Oil Chemistry Society, 50, 159-167, 1973) constitue le test normalisé le plus couramment utilisé pour déterminer la biodégradation d'un composé. Il s'agit d'un test en milieu liquide rapide et facilement réalisable. Il s'agit d'un test conduit en système fermé où le composé à tester est soumis à la biomasse cellulaire constituée par une boue de station d'épuration d'eaux urbaines (STEP). La dégradation est alors mesurée par la production finale de CO₂ piégé par une base. Selon Battersby et al. (Chemosphere, 38, 3219-3235, 1999), le test de Sturm n'est applicable qu'aux produits organiques solubles dans l'eau et ayant une faible volatilité Le test de biodégradabilité correspondant à celui de l'espace de tête en CO₂ a été utilisé pour le 2-EHN. Ce test a montré que le 2-EHN n'était pas facilement biodégradable selon les critères usuels qui imposent 60 % de biodégradation dans les 28 jours avec, au moins, 10 % de biodégradation dans les 10 jours. Selon les auteurs d'un rapport issu de l'American Chemistry Council (2006) concernant le 2-EHN, l'absence de biodégradabilité doit donc être attribuée à la volatilité du substrat. Compte tenu des faibles quantités de substrat mises en oeuvre dans ce test normalisé, le 2-EHN initial qui sert de substrat se trouve principalement localisé dans l'espace de tête de la fiole d'essai et il n'est pas alors disponible pour la microflore. Ce test n'est donc pas non plus pertinent pour le 2-EHN.

Il est connu que les hydrocarbures substitués sont souvent récalcitrants à la biodégradation. Le 2-EHN possède une structure ramifiée, et par conséquent, il n'est pas ou très mal dégradé par les microflores de station d'épuration (STEP) qui sont utilisées classiquement dans les tests de biodégradation.

Nous avons découvert une bactérie permettant de dégrader le 2-EHN et adapté un test permettant de mesurer la biodégradabilité de ce composé.

### Présentation sommaire de l'invention:

La présente invention décrit un procédé de traitement d'effluents hydrocarbonés contenant du 2-EHN dans lequel on fait croître en présence d'un substrat approprié une bactérie *Corynebacterium urealyticum* CIP-I-2126 et on fait dégrader au moins en partie le 2-EHN contenu dans les effluents par la biomasse de ladite bactérie ainsi produite.

Un des objets de l'invention est de décrire un procédé mettant en oeuvre cette bactérie dans le but de dégrader ce composé dans des effluents aqueux de façon que les rejets soient compatibles avec les normes en vigueur.

Un autre objet de l'invention est l'utilisation de cette bactérie pour la décontamination *in situ* des sols et eaux pollués.

### Présentation détaillée de l'invention:

Le procédé de traitement d'effluents hydrocarbonés contenant du 2-EHN selon la présente invention utilise une bactérie déposée à l'Institut Pasteur (Collection Nationale de Cultures des Micro-organismes de l'Institut Pasteur, 25, rue du Docteur Roux, F75724, Paris Cedex 15) sous le nom de *Corynebacterium urealyticum* CIP-I-2126, suite à une identification utilisant le test chimiotaxonomique commercial API Coryne (Biomerieux, Marcy l'Etoile, France). Cependant, la séquence de l'ADNr 16S du micro-organisme (numéro d'accès : AF190800) est identique à celle de la souche de référence de *Mycobacterium austroafricanum.*

La bactérie selon l'invention a été préalablement sélectionnée dans une nappe phréatique qui a été polluée par une essence, avant d'être ensemencée en présence dudit effluent contenant le 2-EHN.

Le milieu de culture utilisé pour la croissance des bactéries est le milieu minéral salin vitaminé (MMSV) et un substrat carboné qui constitue la source de carbone et d'énergie. La source de carbone est introduite soit avant l'autoclavage, soit au moment de l'ensemencement.

Selon une caractéristique du procédé, le substrat de croissance peut comprendre lesdits effluents contenant le 2-EHN.

Selon une variante, le substrat de croissance peut être choisi dans le groupe formé par les hydrocarbures, de 5 à 16 atomes de carbone, les alcools, les mono- ou di- acides carboxyliques sous forme de leurs sels de métal alcalin tels que ceux d'acétate ou de succinate ou encore certains détergents dont la partie hydrophobe est constituée d'une chaîne carbonée linéaire tels que le Tween^{®} 80 (mono-oléate de polyoxyéthylène sorbitan également connu sous le nom de Polysorbate 80, N° CAS 9005-65-6).
Le substrat est préférentiellement choisi parmi le Tween^{®} 80, l'isooctane, l'acétate ou succinate d'un sel de métal alcalin, le glycérol, l'éthanol et leurs mélanges.

Selon un mode préféré, pour améliorer la vitesse de croissance de *Corynebacterium urealyticum* CIP-I-2126 sur 2-EHN, on effectue une préculture, préférentiellement sur Tween^{®} 80, suivie du lavage de cellules, avant d'ensemencer le milieu salin contenant du 2-EHN comme unique substrat de croissance.

On peut également enrichir l'effluent en substrat de croissance, par exemple avec du Tween^{®} 80.
Selon une autre caractéristique du procédé, la concentration en 2-EHN dans les effluents liquides est au plus égale à 12 mg/L et de préférence comprise entre 6 et 10 mg/L.

Les effluents peuvent contenir en plus des hydrocarbures appartenant à la coupe gazole.

Avantageusement, la souche C. *urealyticum* CIP-I-2126 possède la capacité de dégrader le 2-EHN en présence d'une phase liquide hydrophobe non biodégradable servant de réservoir pour le substrat. Cette phase solvant atténue ainsi la concentration solubilisée dans le milieu. Au fur et à mesure de sa consommation, le substrat est transféré de la phase solvant vers le milieu de culture. On évite donc l'inhibition par excès de concentration de substrat. Préférentiellement la phase liquide non biodégradable est choisie parmi le 2,2,4,4,6,8,8-heptaméthylnonane (HMN) et l'huile de silicone.

Le 2-EHN peut être avantageusement utilisé comme source d'azote pour le micro-organisme.

L'évaluation de la biodégradabilité est réalisée par le calcul du taux de dégradation du 2-EHN et du rendement de minéralisation.
Le taux de dégradation du 2-EHN est défini comme la fraction molaire de 2-EHN consommée par la culture.
Le taux de minéralisation est défini comme le rapport entre le nombre de moles finales de carbone dégagées sous forme de CO₂ et le nombre de moles de carbone de 2-EHN introduit.

Les techniques *ex-situ* d'épuration biologique des pollutions concernent les sols et les eaux souterraines. Le traitement des sols fait appel aux techniques des biofiltres conventionnels ou percolants *(trickling biofilters* en langue anglaise). Dans ces biofiltres, les bactéries sont fixées sur un support minéral ou organique ou bien peuvent être ajoutées comme inoculum à des boues de station d'épuration. Les effluents peuvent être enrichis en substrat de croissance (Tween^{®} 80 par exemple) pour améliorer la croissance de la souche.

Selon un mode de réalisation, on peut utiliser la souche selon l'invention, *in situ* dans une biobarrière. Les micro-organismes cultivés en réacteurs sont fixés sur un support solide (granulés de tourbe, plaques perforées en acier inoxydable) placé à l'intérieur d'une tranchée placée sur le trajet du panache pollué, de préférence perpendiculairement au trajet. L'oxygène est apporté par injection d'air dans la tranchée. Le polluant est dégradé lors de son passage à travers la biobarrière.

L'invention sera mieux comprise au vu des exemples suivants qui illustrent l'invention.

### Exemple 1 (selon l'invention)

### Isolement de souches dégradant le 2-EHN

La méthode d'isolement est essentielle pour l'obtention d'une souche dégradant le 2-EHN.
Des échantillons d'eau, prélevés d'une nappe phréatique anciennement polluée par une coupe d'essence, sont utilisés pour ensemencer, au taux d'inoculation de 10% (v/v), des fioles hermétiquement fermées contenant un milieu minimum salin vitaminé (MMSV) dont la composition est la suivante :

| | | |
|---|---|---|
| Na₂HPO₄, 12H₂O | 4,5 | g/L |
| NH₄NO₃ | 1,0 | g/L |
| KH₂PO₄ | 680 | mg/L |
| MgSO₄, 7H₂O | 100 | mg/L |
| FeSO₄, 7H₂O | 1 | mg/L |
| MnSO₄, H₂O | 100 | µg/L |
| (NH₄)₆Mo₇O₂₄, H₂O | 25 | µg/L |

| | | |
|---|---|---|
| NaB₄O₇, 10H₂O | 25 | µg/L |
| Co(NO₃)₂, 6H₂O | 25 | µg/L |
| CuCl₂ | 25 | µg/L |
| ZnCl₂ | 25 | µg/L |
| NH₄NO₃ | 10 | µg/L |
| biotine | 200 | µg/L |
| pyridoxine | 100 | µg/L |
| riboflavine | 50 | µg/L |
| acide nicotinamique | 50 | µg/L |
| panthoténate | 50 | µg/L |
| acide p-aminobenzoïque | 50 | µg/L |
| acide lipoïque | 50 | µg/L |
| acide folique | 20 | µg/L |
| thiamine | 15 | µg/L |
| cyanocobalamine | 1,5 | µg/L |

Immédiatement après ensemencement, le substrat de croissance, un alcane substitué, l'iso-octane (ou 2,2,4-triméthylpentane) est ajouté au milieu MMSV à raison de 500 mg/L. L'isooctane permet avantageusement d'enrichir la population bactérienne en microorganismes capables de dégrader le 2-EHN car c'est un hydrocarbure substitué qui présente des propriétés inhibitrices inférieures à celles du 2-EHN.
Après trois semaines d'incubation sous agitation à 30°C, on constate que l'absorbance de la culture d'enrichissement a augmenté d'environ 0,1 unité. On procède alors à un premier repiquage sur un milieu de composition identique, au même taux d'ensemencement que pour la culture d'enrichissement. Lorsqu'une croissance est à nouveau repérée par augmentation d'absorbance, on effectue un deuxième repiquage dans le mêmes conditions que précédemment.
Après quinze jours d'incubation, une partie aliquote du milieu de culture liquide est alors prélevée et étalée sur des boîtes de Petri de milieu MMSV gélosé. Ces boîtes sont incubées dans une enceinte fermée et de l'iso-octane est fourni sous forme de vapeur en plaçant dans l'enceinte un tube à essais contenant quelques millilitres d'iso-octane pur. Après quinze jours d'incubation à 30°C, des colonies repérées à la surface du milieu gélosé sont isolées. Elles sont capables de dégrader le 2-EHN.

Après une préculture sur Tween^{®} 80 et lavage des cellules, on ensemence le milieu MMSV contenant du 2-éthylhexyl nitrate à raison de 500 mg/L comme unique substrat de croissance.
Après trois semaines d'incubation sous agitation à 30°C, on constate une augmentation de la biomasse cellulaire (94 mg/L) et une production nette de CO₂ (115 mg/L).
Parmi de nombreuses souches testées, seule la souche C. *urealyticum* CIP-I-2126, isolée à partir d'une nappe phréatique contaminée par une essence, est capable de dégrader le 2-EHN dans ces conditions de culture. Il est nécessaire de traiter beaucoup d'échantillons pour isoler une souche active, ce qui indique que ces dernières sont relativement rares dans l'environnement.

### Mise en évidence de la capacité de dégradation pour le 2-EHN

Pour évaluer de façon simple et quantitative la capacité de dégradation du 2-EHN de la souche C. *urealyticum* CIP-I-2126, nous avons utilisé le test suivant :
Une fiole de pénicilline de 120 mL de capacité, hermétiquement fermée et contenant 10 mL de milieu MMSV auquel a été ajouté 486 mg/L de 2-EHN, est ensemencée par la souche CIP-I-2126. La culture est placée sous agitation (150 rpm) à la température de 30°C pendant 28 jours. Le 2-EHN résiduel des fioles est mesuré après extraction au méthyléthyl éther (MTBE) et séparation par chromatographie en phase gazeuse (CPG) sur colonne PONA (Chrompack) en détection à ionisation de flamme.
Parallèlement à la mesure du substrat résiduel, la quantité de CO₂ produite au cours de l'essai est suivie par chromatographie en prélevant des fractions aliquotes (250 µL) de l'espace de tête de la fiole. L'échantillon gazeux prélevé est injecté dans un chromatographe équipé d'une colonne Porapak (Millipore Corp.) et muni d'un détecteur catharométrique. Le CO₂ produit est évalué à l'aide d'un standard externe. On calcule le taux de dégradation du 2-EHN ainsi que le rendement de minéralisation. Les résultats obtenus avec la souche CIP-I-2126 sont présentés dans le tableau 1.

**Tableau 1 : Suivi cinétique de la dégradation du 2-EHN par la souche CIP-I-2126**

| **DURÉE D'INCUBATION (JOURS)** | **TAUX DE DÉGRADATION (%)** |
|---|---|
| 0 | 0 |
| 8 | 35 |
| 10 | 40 |
| 12 | 63 |
| 14 | 70 |
| 15 | 91 |
| 20 | 100 |
| 28 | 100 |

On constate que la souche C. *urealyticum* CIP-I-2126 est capable de dégrader la totalité du 2-EHN introduit. A la fin de l'essai, 20% du carbone ont été minéralisés en CO₂. Le carbone qui n'est pas minéralisé est utilisé notamment pour la production de biomasse cellulaire.

### Identification de la souche CIP-I-2126

La souche CIP-I-2126 qui possède la capacité de dégrader le 2-EHN a été soumise à des tests biochimiques afin d'être identifiée par ses caractères phénotypiques.
La souche CIP-I-2126 est un bacille aérobie strict à Gram positif, non mobile, non sporulé, non capsulé, non ramifié. Les caractères biochimiques de l'identification sont les suivants :
Métabolisme respiratoire aérobie strict
   catalase +
   oxydase -
   nitrate réductase -
   nitrite réductase -
   urée +
   esculine -
   tween-80-estérase +
   Galerie API Coryne caractères positifs : α-glucosidase, urée, catalase.
Les caractères biochimiques exprimés confirment que la souche CIP-I-2126 appartient à l'espèce *Corynebacterium urealyticum.*

### Exemple 2 (comparatif)

Des échantillons de boues activées prélevées dans une station d'épuration d'eaux usées urbaines sont utilisés pour effectuer des isolements de souches dans des conditions identiques à celles décrites dans l'Exemple 1.

Après trois semaines d'incubation, aucun des sept isolats bactériens n'est capable de dégrader le 2-EHN.

### Exemple 3 (effet de la concentration en 2-EHN)

La souche *Corynebacterium urealyticum* CIP-I-2126 est cultivée dans les mêmes conditions de température et d'agitation que dans l'exemple 1, mais en présence des concentrations suivantes de 2-EHN: 238, 486, 1150 et 2310 mg/L. Les taux de dégradation du 2-EHN dans les différents essais sont indiqués dans le tableau 2.

**Tableau 2 : Influence de la concentration en 2-EHN sur la dégradation**

| **TENEUR INITIALE EN 2-EHN (MG/L)** | **DURÉE** | **TAUX DE DÉGRADATION (%)** |
|---|---|---|
| 238 | 28 jours | 100 |
| 486 | 28 jours | 100 |
| 1150 | 28 jours | 50 |
| 2310 | 28 jours | 16 |

Le tableau 2 indique que la souche *C. urealyticum* CIP-I-2126 est capable de dégrader le 2-EHN à plus de 100 % jusqu'à au moins une concentration de 486 mg/L. Par ailleurs, la souche résiste à une concentration de 2,3 g/L de 2-EHN dans le milieu, mais la dégradation est alors incomplète (Tableau 2).

### Exemple 4 (capacité de dégradation en présence d'une phase liquide non biodégradable)

On étudie la capacité de dégradation du 2-EHN par la souche *C. urealyticum* CIP-I-2126 en présence d'une phase liquide hydrophobe non biodégradable servant de réservoir de substrat. Les cultures ont été conduites à 30°C en respiromètre de façon a déterminer les caractéristiques cinétiques de la biodégradation.
Deux phases liquides non biodégradables sont testées : le 2,2,4,4,6,8,8-heptamethylnonane (HMN) et l'huile de silicone (Tableau 3).

**Tableau 3 : Dégradation du 2-EHN par la souche C.urealyticum CIP- I -2126 en présence d'une phase hydrophobe**

| **PHASE LIQUIDE AJOUTÉE** | **TAUX DE DÉGRADATION (%)** | **DURÉE DE BIODÉGRADATION (JOURS)** | **VITESSE MAXIMALE* DE DÉGRADATION (MMOLO₂/J)** |
|---|---|---|---|
| aucune | 100 | 20 | 0,3 |
| HMN | 100 | 10 | 5,3 |
| Huile de silicone | 100 | 10 | 5,8 |

| | | | |
|---|---|---|---|
| * la vitesse maximale de dégradation est exprimée en mmol d'O₂ consommé par jour par fiole contenant 500 mL de milieu à 500 mg/L de 2-EHN. | | | |

Le tableau 3 indique que la souche *C. urealyticum* CIP-I-2126 dégrade encore plus rapidement le 2-EHN lorsque celui-ci est introduit dans une phase liquide non biodégradable. Celle-ci diminue la concentration de 2-EHN à l'équilibre dans la phase aqueuse et diminue l'effet inhibiteur du 2-EHN dissous.

### Exemple 5 : Capacité de dégradation du 2-éthylhexanol, intervenant dans la fabrication du 2-EHN et des mono- et di- 2 éthylhexyl phtalates.

Des tests ont été effectués sur la capacité de dégradation du 2-éthylhexanol par la souche *C. urealyticum* CIP-I-2126 en présence d'une phase liquide hydrophobe non biodégradable servant de réservoir de substrat, le 2,2,4,4,6,8,8-heptamethylnonane (HMN).
Le di-2-éthylhexyl phtalate (DEHP) appartient à la famille des esters d'acide phtalique. Le DEHP est utilisé comme plastifiant dans la chlorure de polyvinyle (PVC) pour apporter aux matériaux flexibilité, résistance et tolérance à la température. De par sa très large utilisation, le DEHP se retrouve , en tant que contaminant, dans l'eau potable, les eaux usées et les sédiments. Dans l'environnement, l'hydrolyse du DEHP fournit de l'acide phatlique dont la biodégradabilité est largement connue et du 2-éthylhexanol. Le 2-éthylhexanol est plus récalcitrant et s'accumule en tant que tel ou dans l'environnement sous forme de son produit d'oxydation, l'acide 2-éthylhexanoïque (Nakamiya et al. J. Biosc. Bioeng. 99, 115-119 (2005) et Chen et al. Appl.Microbiol. Biotech. 74, 676-682, 2007). Or l'hydrolyse du groupement nitrate du 2-EHN peut conduire également à la formation du 2-éthylhexanol. La dégradation de cet intermédiaire a donc été testée.
Les cultures ont été conduites à 30°C en fioles pénicilline, la production de CO₂ a été mesurée par CPG à détection catharométrique. Le 2-éthylhexanol résiduel a été extrait au MTBE puis dosé par CPG/FID après 28 jours de dégradation par la souche. La dégradation du 2-éthylhexanol par la souche C. *urealyticum* CIP-I-2126 est de 100%. Le rendement de minéralisation du 2-éthylhexanol en CO₂ est de 20%.

## Revendications

1. Procédé de traitement d'effluents contenant du 2-éthylhexyl nitrate (2-EHN) dans lequel on fait croître en présence d'un substrat approprié une bactérie *Corynebacterium urealyticum* CIP-1-2126 et on fait dégrader au moins en partie le 2-EHN contenus dans les effluents par la biomasse de ladite bactérie ainsi produite.

2. Procédé selon la revendication 1 dans lequel la bactérie est préalablement sélectionnée dans une nappe phréatique anciennement polluée par une essence avant d'être ensemencée en présence dudit effluent contenant le 2-EHN.

3. Procédé selon l'une des revendications 1 ou 2 dans lequel le substrat comprend lesdits effluents contenant le 2-EHN.

4. Procédé selon l'une des revendications 1 à 3 dans lequel le substrat est choisi dans le groupes formés par les hydrocarbures de 5 à 16 atomes de carbone, les alcools, les mono ou di- acides carboxyliques ou leurs sels de métal alcalin, et leurs mélanges.

5. Procédé selon la revendication 4 dans lequel le substrat est choisi parmi le mono-oléate de polyoxyéthylène sorbitan N° CAS 9005-65-6 l'iso-octane, l'acétate ou succinate d'un sel de métal alcalin, le glycérol, l'éthanol et leurs mélanges.

6. Procédé selon l'une des revendications 1 à 5 dans lequel on effectue une préculture suivie d'un lavage, avant d'ensemencer le milieu salin contenant du 2-EHN comme unique substrat de croissance.

7. Procédé selon l'une des revendications 1 à 6 dans lequel on enrichit le substrat de croissance avec du mono-oléate de polyoxyéthylène sorbitant N°CAS 9005-65-6.

8. Procédé selon l'une des revendications 1 à 7 dans lequel la concentration en 2-EHN dans les effluents liquides est au plus égale à 12 mg/L et de préférence comprise entre 6 et 10 mg/L.

9. Procédé selon l'une des revendications 1 à 8 dans lequel le 2-EHN est dégradé en présence d'une phase liquide non biodégradable servant de réservoir pour le substrat.

10. Procédé selon la revendication 9 dans lequel ladite phase liquide non biodégradable est choisie parmi le 2,2,4,4,6,8,8-heptaméthylnonane (HNM) et l'huile de silicone.

11. Procédé selon l'une des revendications 1 à 10 dans lequel le 2-EHN est utilisé comme source d'azote.

12. Procédé selon l'une des revendications 1 à 11 dans lequel les effluents contiennent une coupe gazole.

13. Procédé selon l'une des revendications 1 à 12 dans lequel on fait développer lesdites bactéries sur un système d'au moins un biofiltre, on introduit les effluents dans le biofiltre et on soutire l'effluent débarrassé au moins en partie du 2-EHN.

14. Procédé selon la revendication 13, dans lequel les bactéries sont fixées sur un support minéral ou organique, ou sont ajoutées comme inoculum à des boues de station d'épuration.

15. Procédé selon l'une des revendications 1 à 12 dans lequel on fait développer lesdites bactéries sur un support solide placé à l'intérieur d'une tranchée perpendiculaire au trajet de l'effluent pollué constituant une biobarrière, on introduit de l'oxygène par injection d'air dans la tranchée, le polluant étant dégradé lors de son passage à travers la biobarrière.

## Patentansprüche

1. Verfahren zur Behandlung von Abwässern, die 2-Ethylhexylnitrat (2-EHN) enthalten, wobei man in Gegenwart eines geeigneten Substrats eine Bakterie, Corynebacterium urealyticum CIP-I-2126, wachsen lässt und man das 2-EHN, das in den Abwässern enthalten ist, mindestens teilweise durch die auf diese Weise produzierte Biomasse der Bakterie abbauen lässt.

2. Verfahren nach Anspruch 1, wobei die Bakterie vorher ausgewählt wird aus einem Grundwasser, das früher mit einem Benzin verschmutzt wurde, bevor es in Gegenwart des Abwassers, das das 2-EHN enthält, beimpft wird.

3. Verfahren nach einem der Ansprüche 1 oder 2, wobei das Substrat die Abwässer, die das 2-EHN enthalten, umfasst.

4. Verfahren nach einem der Ansprüche 1 bis 3, wobei das Substrat ausgewählt ist aus den Gruppen gebildet aus Kohlenwasserstoffen mit 5 bis 16 Kohlenstoffatomen, Alkoholen, Mono- oder Dicarbonäuren oder deren Alkalimetallsalzen und deren Gemischen.

5. Verfahren nach Anspruch 4, wobei das Substrat ausgewählt ist aus Polyoxyethylen-sorbitan-monooleat, CAS Nr. 9005-65-6, Isooctan, Alkalimetallsalzsuccinat oder -acetat, Glycerin, Ethanol und deren Gemischen

6. Verfahren nach einem der Ansprüche 1 bis 5, wobei man eine Vorkultur, gefolgt von einer Wäsche, durchführt, bevor das salzige Medium, das 2-EHN als einziges Wachstumssubstrat enthält, beimpft wird.

7. Verfahren nach einem der Ansprüche 1 bis 6, wobei man das Wachstumssubstrat mit Polyoxyethylen-sorbitan-monooleat, CAS Nr. 9005-65-6, anreichert.

8. Verfahren nach einem der Ansprüche 1 bis 7, wobei die Konzentration an 2-EHN in den flüssigen Abwässern höchstens gleich 12 mg/l ist und bevorzugt im Bereich zwischen 6 und 10 mg/l liegt.

9. Verfahren nach einem der Ansprüche 1 bis 8, wobei das 2-EHN in Gegenwart einer nicht biologisch abbaubaren, flüssigen Phase abgebaut wird, die als Reservoir für das Substrat dient.

10. Verfahren nach Anspruch 9, wobei die nicht biologisch abbaubare, flüssige Phase ausgewählt wird aus 2,2,4,4,6,8,8-Heptamethylnonan (HNM) und Silikonöl.

11. Verfahren nach einem der Ansprüche 1 bis 10, wobei das 2-EHN als Stickstoffquelle verwendet wird.

12. Verfahren nach einem der Ansprüche 1 bis 11, wobei die Abwässer einen Dieselschnitt enthalten.

13. Verfahren nach einem der Ansprüche 1 bis 12, wobei man sich die Bakterien auf einem System aus mindestens einem Biofilter entwickeln lässt, man die Abwässer in den Biofilter einführt und man das mindestens teilweise von 2-EHN befreite Abwasser abzieht.

14. Verfahren nach Anspruch 13, wobei die Bakterien auf einem mineralischen oder organischen Träger fixiert werden oder als Inokulum zu Klärschlämmen gegeben werden.

15. Verfahren nach einem der Ansprüche 1 bis 12, wobei man sich die Bakterien auf einem festen Träger entwickeln lässt, der im Innern eines Grabens senkrecht zum Ablaufweg des verschmutzten Abwassers angeordnet ist, der eine Biobarriere bildet, man Sauerstoff durch Injektion von Luft in den Graben einführt, wobei der Schadstoff während seines Durchgangs durch die Biobarriere abgebaut wird.

## Claims

1. A method for treating effluents comprising 2-ethylhexyl nitrate (2-EHN), wherein a *Corynebacterium urealyticum* CIP-I-2126 bacterium is grown in the presence of a suitable substrate and the 2-EHN contained in the effluents is at least partly degraded by the biomass of said bacterium thus produced.

2. A method as claimed in claim 1, wherein the bacterium is first selected in ground water formerly polluted by a gasoline prior to being seeded in the presence of said 2-EHN-containing effluent.

3. A method as claimed in any one of claims 1 or 2, wherein the substrate comprises said 2-EHN-containing effluents.

4. A method as claimed in any one of claims 1 to 3, wherein the substrate is selected from the group consisting of hydrocarbons with 5 to 16 carbon atoms, alcohols, carboxylic mono- or diacids or their alkali metal salts, and mixtures thereof.

5. A method as claimed in claim 4, wherein the substrate is selected from polyoxyethylene sorbitan monooleate CAS No. 9005-65-6, iso-octane, acetate or succinate of an alkali metal salt, glycerol, ethanol and mixtures thereof.

6. A method as claimed in any one of claims 1 to 5, wherein a preculture is performed, followed by cell washing, prior to seeding the saline medium containing 2-EHN as the single growth substrate.

7. A method as claimed in any one of claims 1 to 6, wherein the growth substrate is enriched with polyoxyethylene sorbitan monooleate CAS No. 9005-65-6.

8. A method as claimed in any one of claims 1 to 7, wherein the 2-EHN concentration in the liquid effluents is at most 12 mg/L and it preferably ranges between 6 and 10 mg/L.

9. A method as claimed in any one of claims 1 to 8, wherein the 2-EHN is degraded in the presence of a non-biodegradable liquid phase used as a reservoir for the substrate.

10. A method as claimed in claim 9, wherein said non-biodegradable liquid phase is selected from among 2,2,4,4,6,8,8-heptamethylnonane (HNM) and silicone oil.

11. A method as claimed in any one of claims 1 to 10, wherein the 2-EHN is used as a nitrogen source.

12. A method as claimed in any one of claims 1 to 11, wherein the effluents contain a gas oil cut.

13. A method as claimed in any one of claims 1 to 12, wherein said bacteria are developed on a system of at least one biofilter, the effluents are fed into the biofilter and the effluent at least partly freed of 2-EHN is discharged.

14. A method as claimed in claim 13, wherein the bacteria are attached to a mineral or organic support, or they are added as an inoculum to sewage plant sludge.

15. A method as claimed in any one of claims 1 to 12, wherein said bacteria are developed on a solid support arranged inside a trench perpendicular to the route of the polluted effluent forming a biobarrier, and oxygen is provided by injection of air into the trench, the pollutant being degraded as it passes through the biobarrier.
